# EUROPÄISCHE PATENTSCHRIFT

(11) **EP 1 456 159 B1**
(45) Veröffentlichungstag und Bekanntmachung des Hinweises auf die Patenterteilung: **02.08.2006**
(21) Anmeldenummer: 02792927.2
(22) Anmeldetag: 06.12.2002
(51) Int. Cl.: C07C 29/80, C07C 31/22

(54) **VERFAHREN ZUR ISOLIERUNG VON TRIMETHYLOLPROPAN AUS EINEM REAKTIONSGEMISCH**
METHOD FOR THE ISOLATION OF TRIMETHYLOL PROPANE FROM A REACTION MIXTURE
PROCEDE D'ISOLATION DE TRIMETHYLPROPANE A PARTIR D'UN MELANGE DE REACTION

(30) Priorität: 07.12.2001 DE 10160180
(43) Veröffentlichungstag der Anmeldung: 15.09.2004
(73) Patentinhaber: BASF Aktiengesellschaft, 67056 Ludwigshafen (DE)
(72) Erfinder: SIRCH, Tilman, 67105 Schifferstadt (DE); KAIBEL, Gerd, 68623 Lampertheim (DE); WARTINI, Alexander, 69120 Heidelberg (DE); DERNBACH, Matthias, 69221 Dossenheim (DE)
(74) Vertreter: Reitstötter - Kinzebach
(86) Internationale Anmeldenummer: PCT/EP2002/013882
(87) Internationale Veröffentlichungsnummer: WO 2003/048093

(56) Entgegenhaltungen:
- EP-A- 1 013 631
- DE-A- 19 963 435

## Beschreibung

Die vorliegende Erfindung betrifft ein Verfahren zur Isolierung von Trimethylolpropan aus einem Reaktionsgemisch, das durch Umsetzung von n-Butyraldehyd mit Formaldehyd in Gegenwart einer Base und gegebenenfalls anschließende Hydrierung erhalten wird.

Trimethylolpropan, das im folgenden auch als TMP abgekürzt wird, ist ein dreiwertiger Alkohol, der im großen Umfang zur Herstellung von Lacken, Polyurethanen und Polyestern verwendet wird. TMP wird durch Aldolkondensation von n-Butyraldehyd mit Formaldehyd hergestellt. Je nach Verfahrensführung unterscheidet man zwischen verschiedenen Varianten.

Beim sogenannten Cannizzaro-Verfahren wird Butyraldehyd unter Zugabe einer stöchiometrischen Menge Base mit Formaldehyd umgesetzt. Dabei steht zunächst 2,2-Dimethylolbutanal, das dann in einer gekreuzten Cannizzaro-Reaktion mit einem weiterem Molekül Formaldehyd zu Ameisensäure und Trimethylolpropan reagiert. Der Nachteil dieser Verfahrensweise besteht darin, daß sich als Koppelprodukt ein Moläquivalent Ameisensäuresalz bildet, das aufgearbeitet werden muss und den Verbrauch an Formaldehyd erhöht.

Das sogenannte Hydrierverfahren stellt eine Weiterentwicklung dar. Dabei wird Butyraldehyd in Gegenwart einer katalytischen Menge eines tertiären Amins mit Formaldehyd umgesetzt. Die Reaktion bleibt auf der Stufe des 2,2-Dimethylolbutanals stehen, das anschließend zu Trimethylolpropan hydriert wird. Es fallen keine stöchiometrischen Mengen an Ameisensäuresalzen an und die erhaltene Lösung ist leichter aufzuarbeiten, da weniger störende Nebenprodukte entstehen. Ein derartiges Verfahren ist zum Beispiel in der WO 98/28253 beschrieben. Bei diesem Verfahren werden, um eine möglichst vollständige Umsetzung der Edukte zu Dimethylolbutanal zu erreichen, nicht umgesetzte oder teilumgesetzte Ausgangsmaterialien vom Reaktionsgemisch abgetrennt und in die Aldolisierungsstufe zurückgeführt; der Rückstand wird katalytisch und/oder thermisch behandelt, wobei sich Monomethylolbutanal in Dimethylolbutanal und Ethylacrolein umwandelt und letzteres abgetrennt und in die Aldolisierungsstufe zurückgeführt werden kann. Das nach dem Verfahren erhaltene Roh-TMP soll in üblicher Weise destillativ aufgearbeitet werden.

Die DE 199 63 435 beschreibt ein Verfahren zur Reindestillation von Roh-TMP, das nach dem Hydrierverfahren erhalten wird, bei dem man zunächst Wasser, Methanol, Trialkylamin und/oder Trialkylammoniumformiat destillativ abtrennt, den erhaltenen Rückstand auf eine Temperatur erwärmt, bei der TMP flüchtig ist und höher als TMP siedende Verbindungen zumindest teilweise gespalten werden, wobei TMP und leichter als TMP siedende Verbindungen destillativ abgetrennt werden; und das erhaltene Destillat unter Abtrennung der leichter flüchtigen Verbindungen und Gewinnung von reinem TMP destilliert wird. Das erhaltene TMP kann dann nochmals einer Reindestillation unterzogen werden.

Bei diesem Verfahren ist es jedoch von Nachteil, daß neben den Leichtsiedern auch TMP über Kopf gezogen werden muss und dadurch ein hoher Energiebedarf entsteht. Außerdem werden bei der Destillation in aufeinanderfolgenden Kolonnen, begünstigt durch hohe Verweilzeiten in den Kolonnensümpfen, deutliche Mengen unerwünschter Nebenprodukte gebildet, die die Produktausbeute mindern. So tritt bei der Abtrennung der leichter als TMP flüchtigen Verbindungen - obgleich es sich bei dem zu trennenden Gemisch um das Kopfprodukt der vorausgegangenen Destillation handelt - erneut ein Hochsiederanteil auf. Dieser kann zwar zurückgeführt und teilweise thermisch gespalten werden, aber es ist unwirtschaftlich, eine größere Menge an Hochsiedern auf diese Weise im Kreis zu führen. Die Errichtung und der Betrieb mehrerer Kolonnen stellen darüber hinaus einen wesentlichen Kostenfaktor dar.

Der Erfindung liegt daher die Aufgabe zugrunde, ein Verfahren zur Isolierung von Trimethylolpropan aus einem Reaktionsgemisch bereitzustellen, das bei geringen Investitionskosten und geringem Energiebedarf TMP hoher Reinheit liefert.

Die Aufgabe wird erfindungsgemäß gelöst durch ein Verfahren, dem man
i) das Gemisch in eine Zulaufsäule mit oberhalb der Zulaufstelle gelegenem Verstärkungsteil und unterhalb der zulaufstelle gelegenem Abtriebsteil einführt,
ii) eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Aufheizer am unteren Säulenende vorsieht,
iii)eine mit der oberen Vereinigungssäule und der unteren Vereinigungssäule kommunizierende Abzugssäule vorsieht,
iv) aus der Abzugssäule reines Trimethylolpropan als Seitenabzug abzieht, und am Kopf oder im oberen Bereich der oberen Vereinigungssäule leichter als Trimethylolpropan siedende Verbindungen und im Sumpf oder im unteren Bereich der unteren Vereinigungssäule höher als Trimethylolpropan siedende Verbindungen abzieht.

Das erfindungsgemäße Verfahren eignet sich sowohl zur Aufarbeitung von Reaktionsgemischen, die nach dem Cannizzaro-Verfahren, d.h. in Gegenwart einer stöchiometrischen Menge Base, erhalten werden, als auch solcher, die nach dem Hydrierverfahren erhalten werden, d.h. in Gegenwart einer katalytischen Menge eines tertiären Amins und anschließende Hydrierung.

Die Zulaufsäule, Abzugssäule, obere Vereinigungssäule und untere Vereinigungssäule können diskrete Bauelemente sein oder als Abschnitt oder Kammer einer Kolonne ausgebildet sein, die mehrere Funktionen vereint. Der Ausdruck "kommunizierende Säulen" bedeutet, daß zwischen ihnen ein Austausch sowohl von aufsteigenden Brüden als auch von ablaufendem Kondensat erfolgt.

In einer bevorzugten Ausführungsform verwendet man zur Destillation eine Trennwandkolonne, das heißt die Zulaufsäule und die Abzugssäule sind als sich über einen Abschnitt der Längsausdehnung einer Kolonne erstreckende, beidseitig zu je einem Vereinigungsraum geöffnete Teilkammern ausgebildet, die durch eine Trennwand voneinander getrennt sind. Destillationskolonnen, die eine Trennwand enthalten, sind an sich bekannt und z. B. beschrieben in ECN 2-8 Oktober 1995, S. 26, "BASF Distils Energie Savings"; Chem. Eng. Res. Des. (1993) 71(3), S. 307, "The control of dividing wall column"; US 2,471,134, Chem. Eng. Res. Des., Part A: Trans IChemE, Sept. 1993, S. 639-644, "Heat transfer across the wall of dividing wall".

In alternativen Ausführungsformen verwendet man zur Destillation eine Destillationskolonne mit einer thermisch gekoppelten Vorkolonne, das heißt die Abzugssäule, die obere Vereinigungssäule und die untere Vereinigungssäule sind als einstückige Destillationskolonne ausgebildet und die Zulaufsäule ist als Vorkolonne zur Destillationskolonne ausgebildet. Alternativ verwendet man eine Destillationskolonne mit einer thermisch gekoppelten Nachkolonne, das heißt die Zulaufsäule, die obere Vereinigungssäule und die untere Vereinigungssäule sind als einstückige Destillationskolonne ausgebildet und die Abzugssäule ist als Nachkolonne zu der Destillationskolonne ausgebildet. Destillationskolonnen mit beigeschalteten Hilfskolonnen sind bekannt und z. B. in Chem. Eng. Res. Des., Part A: Trans IChemE, März 1992, S. 118-132, "The design and optimisation of fully thermally coupled distillation columns" beschrieben.

Der besondere Vorteil beim vorliegenden Verfahren resultiert unter anderem daraus, daß die Rein-TMP-Fraktion im Seitenabzug abgeführt werden kann und nicht mehr über Kopf getrieben werden muss. Dadurch können gegenüber bekannten Verfahren erhebliche Energieeinsparungen bei ansonsten unveränderter Qualität erzielt werden. Darüber hinaus vermindert eine unter vergleichsweise produktschonenden Bedingungen erreichbare Reduzierung der Verweilzeit von Wertprodukt im heißen Kolonnensumpf die ausbeuteschädliche Bildung unerwünschter Nebenprodukte. Die DE 199 634 35 konnte das erfindungsgemäße Verfahren nicht nahe legen, da es ein wesentliches Merkmal des dort gelehrten Verfahrens ist, den nach Abtrennung von Wasser, Methanol, Trialkylamin- und/oder Trialkylammoniumformiat erhaltenen Rückstand auf eine Temperatur zu erhitzen, bei der TMP flüchtig ist und höher als TMP siedende Verbindungen zumindest teilweise gespalten werden.

Reaktionsgemische, die nach dem erfindungsgemäßen Verfahren aufgearbeitet werden können, enthalten neben TMP üblicherweise Wasser, Methanol, einwertige Alkohole wie Methylbutanol und mehrwertige Alkohole, z. B. Diole wie Ethylpropandiol, sowie Acetale von Formaldehyd und Methanol an TMP und Ether von einwertigen Alkoholen und mehrwertigen Alkoholen, z. B. Diolen, sowie gegebenenfalls tertiäre Ammoniumformiate. Ein nach dem Hydrierverfahren erhaltenes Reaktionsgemisch hat üblicherweise folgende Zusammensetzung: 10 bis 40 Gew.-% Trimethylolpropan, 0,5 bis 5 Gew.-% Methanol, 1 bis 6 Gew.-% einwertige Alkohole, 1 bis 10 Gew.-% tertiäre Ammoniumformiate, 0 bis 5 Gew.-% Diole, 2 bis 10 Gew.-% Hochsieder sowie 50 bis 80 Gew.-% Wasser.

Zur TMP-Herstellung nach dem Hydrierverfahren setzt man im Allgemeinen Butyraldehyd mit 2 bis 8 Mol, vorzugsweise 2 bis 3,5 Mol Formaldehyd um. Das tertiäre Amin wird in der Regel in einer Menge von 0,001 bis 0,2, vorzugsweise 0,01 bis 0,07 Moläquivalenten, bezogen auf Butyraldehyd eingesetzt. Geeignete Amine sind Trialkylamine wie z.B. Trimethylamin oder Triethylamin. Die Reaktion wird im Allgemeinen bei einer Temperatur von 5 bis 100 °C, vorzugsweise 15 bis 80 °C durchgeführt. Die Verweilzeit wird - in Abhängigkeit von der Temperatur - in der Regel auf 0,25 bis 12 Stunden eingestellt. Mit besonderem Vorteil wird das in der WO 98/28253 beschriebene Verfahren eingesetzt. Das bei der Aldolisierung erhaltene, Dimethylolbutanal enthaltene Produkt wird dann unter üblichen Bedingungen in Gegenwart von Wasserstoffgas hydriert. Als Hydrierkatalysatoren eignen sich insbesondere Kupfer-haltige Trägerkatalysatoren wie sie z.B. in der WO 95/32171 beschrieben sind. Die Hydrierung erfolgt zweckmäßiger Weise kontinuierlich, z.B. in einem mit einer Katalysatorschüttung gefüllten Reaktorrohr, bei dem die Reaktionslösung über die Katalysatorschüttung in Rieselfahrweise oder Sumpffahrweise gepumpt wird.

Es hat sich als günstig erwiesen, aus dem Reaktionsgemisch vor dem Einführen in die Zulaufsäule die Hauptmenge an enthaltenem Wasser zu entfernen, vorzugsweise auf einen Restwassergehalt von weniger als 5 Gew.-%, insbesondere weniger als 2 Gew.-%. Bei der Entwässerung können andere im Reaktionsgemisch enthaltene Leichtsieder, wie Methanol und tertiäres Amin oder tertiäres Ammoniumformiat mit abgetrennt werden. Auf diese Weise kann die anschließende Destillation bei einem geringen Druck durchgeführt werden, ohne dass der Dampfdruck von Wasser störend wirkt. Die Entfernung des Wassers erfolgt vorzugsweise destillativ und oder durch Entspannung. Hierzu sind übliche Verdampfer und/oder Destillationskolonnen geeignet; die Wasserabtrennung kann einstufig oder mehrstufig durchgeführt werden. Sie kann bei einem Druck von 20 mbar bis Umgebungsdruck durchgeführt werden. Verwendet man niedrige Drücke im Bereich von 20 bis 100 mbar und Apparate mit kurzer Verweilzeit, wie z. B. Dünnschichtverdampfer, Fallfilmverdampfer oder Wendelrohrverdampfer, wird in der Regel das tertiäre Ammoniumformiat mit den Leichtsiedern abdestilliert. Bei Drückenvon mehr als 100 mbar findet in dem Verdampfer bzw. dem Sumpf der Destillationskolonne eine Umsetzung von tertiärem Ammoniumformiat mit TMP zu tertiärem Amin und TMP-formiat statt. Das tertiäre Amin destilliert mit den Leichtsiedern ab. Es kann von dem mitabgetrennten Methanol und Wasser destillativ gereinigt und erneut für die Aldolisierung verwendet werden.

Wird die Entwässerung des Reaktionsgemisches unter Bedingungen durchgeführt, bei denen sich in größerem Umfang TMP-formiat bildet, kann es zweckmäßig sein, das Reaktionsgemisch vor dem Einführen in die Zulaufkolonne einer Behandlung zu unterziehen, bei der das TMP-formiat gespalten und TMP zurückgewonnen wird. Dies kann beispielsweise in an sich bekannter Weise dadurch erfolgen, daß man eine Umesterung mit einem von TMP verschiedenen Alkohol, insbesondere einem C₁-C₄-Alkanol, vorzugsweise Methanol, unter Bildung des Formiats dieses Alkohols und TMP durchführt. Die Umesterung kann thermisch oder basisch katalysiert werden. Sie kann gemäß Beschreibung der EP-A 289 921 in Gegenwart katalytischer Mengen von Alkali- oder Erdalkalialkoholaten oder gemäß der Beschreibung der WO 97/17313 in Gegenwart eines tertiären Amins, vorzugsweise Triethylamin, durchgeführt werden. Die Umesterung erfolgt vorzugsweise bei einer Temperatur von 150 bis 250 °C, insbesondere 180 bis 220 °C. Der Katalysator wird, falls verwendet, in der Regel in einer Menge von 0,005 bis 0,05 Mol pro Mol TMP-formiat eingesetzt. Die Reaktionsdauer beträgt typischerweise bis zu zehn Stunden. Zur Erreichung einer angemessenen Reaktionsdauer kann ein separater Reaktor oder Verweilzeitbehälter eingesetzt werden.

Das gegebenenfalls entwässerte, gegebenenfalls einer Umesterung unterzogene Reaktionsgemisch wird dann zur Reindestillation in eine Zulaufsäule eingeführt, die wie beschrieben mit einer oberen und unteren Vereinigungssäule kommuniziert, die ihrerseits mit einer Abzugssäule kommunizieren. Bei der Reindestillation werden höher als Trimethylolpropan siedende Verbindungen (Schwersieder, insbesondere TMP-Acetale) und leichter als Trimethylolpropan siedende Verbindungen abgetrennt. Wurde das Reaktionsgemisch einer Entwässerung unterworfen, wurde bereits die Hauptmenge der Leichtsieder entfernt und in der Reindestillation werden im wesentlichen sogenannte Mittelsieder, das heißt Verbindungen, die zwischen Wasser und TMP sieden, abgetrennt.

Die Reindestillation erfolgt üblicherweise bei einem Druck im Bereich von 5 bis 100 mbar, vorzugsweise 5 bis 50 mbar, insbesondere 10 bis 30 mbar. Das Rücklaufverhältnis beträgt im Allgemeinen 0 bis 30, vorzugsweise 0,2 bis 1.

Die Zulaufsäule, die obere Vereinigungssäule, die untere Vereinigungssäule und die Abzugssäule enthalten trennwirksame Einbauten. Vorzugsweise liegen die Einbauten im Form geordneter Packungen, wie z. B. Sulzer Mellapak, Sulzer BX, Montz A1 oder Montz A3 oder Kühni Rhombopak, oder regelloser Schüttungen von Füllkörpern, wie z. B. Dixon-Ringen, Raschig-Ringen, High-Flow-Ringen oder Raschig-Super-Ringen, vor.

Das Reaktionsgemisch wird vorzugsweise etwa in der Mitte der Zulaufsäule zugeführt. Das reine Trimethylolpropan wird vorzugsweise etwa in der Mitte der Abzugssäule als Seitenabzug gasförmig oder flüssig, vorzugsweise jedoch gasförmig, abgezogen. Das reine TMP weist in der Regel eine Reinheit von wenigstens 98,5 Gew.-% und eine Farbzahl von 0 bis 200 APHA (Bestimmung nach DIN ISO 6271; ASTM-D 1209-93) auf. Ein Strom der sich im Sumpf sammelnden Hochsiedern wird ausgeschleust, um ein Aufpegeln höher siedender und/oder farbgebender Komponenten zu verhindern. Im Sumpf stellt sich im Allgemeinen ein stationärer Gehalt an TMP von 1 bis 50 Gew.-% ein. Am Kopf werden Leicht- und Mittelsieder abgezogen, die 0 bis 80 Gew.-% TMP enthalten.

Das erfindungsgemäße Verfahren wird durch die beigefügten Figuren und das nachfolgende Beispiel näher veranschaulicht.
- Fig. 1: zeigt eine zur Durchführung des erfindungsgemäßen Verfahrens geeignete Anlage, die eine Trennwandkolonne aufweist.
- Fig. 2: zeigt eine zur Durchführung des erfindungsgemäßen Verfahrens geeignete Anlage, die eine Destillationskolonne mit vorgeschalteter Vorkolonne aufweist.

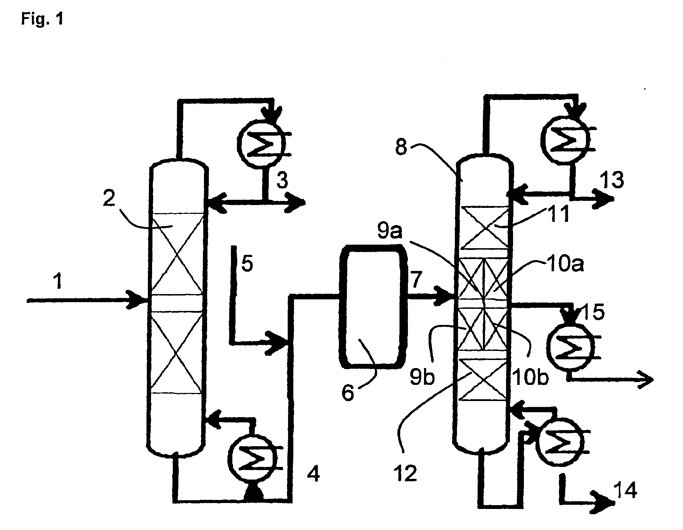

Gemäß Fig. 1 wird das TMP enthaltende Reaktionsgemisch in die Leichtsiederkolonne 2 eingeführt, in der ein Gemisch 3 aus Wasser und Leichtsiedern wie Methanol oder tertiärem Amin abgetrennt wird. Der der Leichtsiederkolonne 2 entnommene Sumpf 4, der TMP, Hochsieder und die nicht in der Leichtsiederkolonne 2 abgetrennten Leichtsieder enthält, wird in den Reaktor 6 eingeführt, in dem TMP-formiat gespalten wird. Dies geschieht durch Zugabe eines Alkohols, wie z. B. Methanol, über Leitung 5. Die Zugabe des Alkohols 5 und Verwendung des Reaktors 6 ist fakultativ. Das behandelte Reaktionsgemisch 7 gelangt in die Trennwandkolonne 8, die aus einer Zulaufsäule 9 mit Verstärkungsteil 9a und Abtriebsteil 9b, einer Abzugssäule 10 mit Abtriebsteil 10a und Verstärkungsteil 10b sowie einer oberen Vereinigungssäule 11 und einer unteren Vereinigungssäule 12 besteht. Am Kopf der Kolonne 8 destillieren niedrigere als TMP siedende Verbindungen 13 ab. Es verbleibt ein Sumpf 14 der reich an Hochsiedern ist. Als Seitenabzug der Abzugssäule wird reines TMP 15 entnommen.

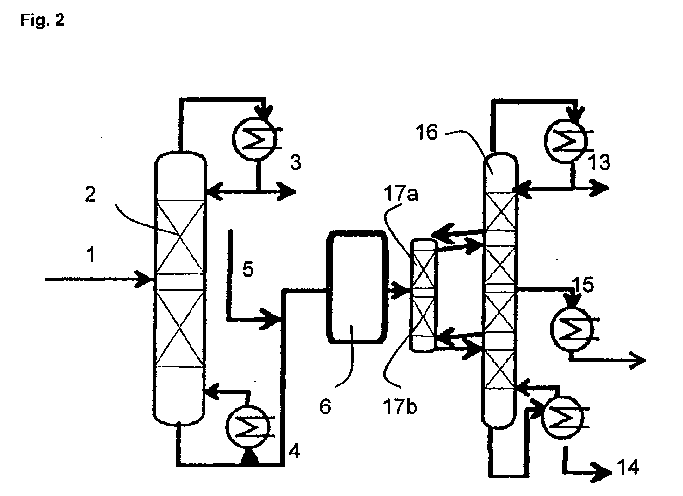

In Fig. 2 haben gleiche Bezugszeichen die gleiche Bedeutung wie in Fig. 1. Im Unterschied zur Anlage gemäß Fig. 1 verwendet man an Stelle der Trennwandkolonne 8 eine Anordnung einer Destillationskolonne 16 mit thermisch gekoppelter Vorkolonne 17. Die Vorkolonne 17 verfügt über einen Verstärkungsteil 17a und einen Abtriebsteil 17b.

### Beispiel:

Ein Reaktionsgemisch, das durch Umsetzung von n-Butyraldehyd mit Formaldehyd in Gegenwart von Trimethylamin und Hydrierung des erhaltenen Gemisches gemäß Bsp. 5 der WO 98/28253 erhalten worden ist und 23,1 Gew.-% TMP, 0,4 Gew.-% Methanol, 0,87 Gew.-% Trimethylamin (als Formiat), 0,97 Gew.-% Methylbutanol, 0,47 Gew.-% Ethylpropandiol, 1,07 Gew.-% Hochsieder und 71,6 Gew.-% Wasser enthielt, wurde in einem Massenstrom von 5 kg/Stunde einer Leichtsiederkolonne zugeführt und bei 400 mbar und 175 °C Sumpftemperatur entwässert. Der Zulauf erfolgte in die Mitte der Kolonne. Verstärkungs- und Abtriebsteil bestanden aus je 1 m Blechpackung (Nennweite NW 50 Kühni Rhombopak 9M). Das Rücklaufverhältnis betrug 0,33. Aus dem Kolonnensumpf wurde ein Strom von 1,1 kg/Stunden abgezogen, der neben Hochsiedern 80 Gew.-% TMP und etwa 1 Gew.-% Wasser enthielt.

100 g/Stunde des Kolonnenaustrags wurden zur Reindestillation einer Trennwandkolonne (NW 100, oberes Vereinigungsteil 0,1 m Dixonringe, unteres Vereinigungsteil 0,3 m Dixonringe, Trennwand mittig, auf der Zulaufseite 0,6 Dixonringe oberhalb des Zulaufs und 0,4 m Dixonringe unterhalb des Zulaufs; auf der Abzugsseite 0,5 m Dixonringe oberhalb des Abzugs und 0,5 m Dixonringe unterhalb des Abzugs) eingeleitet. Die Reindestillation erfolgt über 20 mbar Kopfdruck, einem Rücklaufverhältnis von 0,5 und einem temperaturgeregelten Sumpfaustrag.

Über den Kolonnensumpf wurden Hochsieder mit einem Restgehalt von 12 Gew.-% TMP abgetrennt. Über Kopf wurden die Mittelsieder, insbesondere 2-Ethylpropandiol (5,5 Gew.-%), mit einem Restgehalt von 66 Gew.-% TMP abgezogen. Über den Seitenabzug wurde TMP mit einer Reinheit von 99,0 Gew.-% und einer Farbzahl von weniger 30 APHA abgezogen. Die Ausbeute der Reindestillation betrug insgesamt 79 Gew.-%.

## Patentansprüche

1. Verfahren zur Isolierung von Trimethylolpropan aus einem Reaktionsgemisch, das durch Umsetzung von n-Butyraldehyd mit Formaldehyd in Gegenwart einer Base und gegebenenfalls Hydrierung erhalten ist, durch Destillation, wobei man
i) das Gemisch in eine Zulaufsäule mit oberhalb der Zulaufstelle gelegenem Verstärkungsteil und unterhalb der Zulaufstelle gelegenem Abtriebsteil einführt,
ii) eine mit dem oberen Ende des Verstärkungsteils kommunizierende obere Vereinigungssäule mit Kondensator am oberen Säulenende und eine mit dem unteren Ende des Abtriebsteils kommunizierende untere Vereinigungssäule mit Aufheizer am unteren Säulenende vorsieht,
iii) eine mit der obereren Vereinigungssäule und der unteren Vereinigungssäule kommunizierende Abzugssäule vorsieht,
iv) aus der Abzugssäule reines Trimethylolpropan als Seitenabzug abzieht, und am Kopf oder im oberen Bereich der oberen Vereinigungssäule leichter als Trimethylolpropan siedende Verbindungen und im Sumpf oder im unteren Bereich der unteren Vereinigungssäule höher als Trimethylolpropan siedende Verbindungen abzieht.

2. Verfahren nach Anspruch 1, wobei das Reaktionsgemisch durch Umsetzung von n-Butyraldehyd mit Formaldehyd in Gegenwart einer katalytischen Menge eines tertiären Amins und anschließende Hydrierung erhalten ist.

3. Verfahren nach Anspruch 1 oder 2, wobei man aus dem Reaktionsgemisch vor dem Einführen in die Zulaufsäule die Hauptmenge an enthaltenem Wasser entfernt.

4. Verfahren nach Anspruch 3, wobei man das entwässerte Reaktionsgemisch vor dem Einführen in die Zulaufsäule mit einem von Trimethylolpropan verschiedenen Alkohol versetzt.

5. Verfahren nach einem der vorhergehenden Ansprüche, wobei die Zulaufsäule und die Abzugssäule als sich über einen Abschnitt der Längsausdehnung einer Kolonne erstreckende, beidseitig zu je einem Vereinigungsraum offene Teilkammern, die durch eine Trennwand voneinander getrennt sind, ausgebildet sind.

6. Verfahren nach Anspruch 1 bis 4, wobei die Abzugssäule, die obere Vereinigungssäule und die untere Vereinigungssäule als einstückige Destillationskolonne ausgebildet sind und die Zulaufsäule als thermisch gekoppelte Vorkolonne zu der Destillationskolonne ausgebildet ist.

7. Verfahren nach Anspruch 1 bis 4, wobei die Zulaufsäule, die obere Vereinigungssäule und die untere Vereinigungssäule als einstückige Destillationskolonne ausgebildet sind und die Abzugssäule als thermisch gekoppelte Nachkolonne zu der Destillationskolonne ausgebildet ist.

8. Verfahren nach einem der vorhergehenden Ansprüche, wobei die Zulaufsäule, die obere Vereinigungssäule, die untere Vereinigungssäule und die Abzugssäule trennwirksame Einbauten in Form geordneter Packungen und/oder regelloser Schüttungen von Füllkörpern enthalten.

9. Verfahren nach einem der vorhergehenden Ansprüche, wobei man bei einem Druck im Bereich 5 bis 100 mbar destilliert.

## Claims

1. A process for isolating trimethylolpropane from a reaction mixture which has been obtained by reaction of n-butyraldehyde with formaldehyde in the presence of a base and, if appropriate, hydrogenation by distillation, wherein
i) the mixture is introduced into a feed column having an enrichment section located above the feed point and a stripping section located below the feed point,
ii) an upper combining column which has a condenser at the upper end of the column and communicates with the upper end of the enrichment section and a lower combining column which has a heater at the lower end of the column and communicates with the lower end of the stripping section are provided,
iii) an offtake column which communicates with the upper combining column and the lower combining column is provided,
iv) pure trimethylolpropane is taken off from the offtake column at a side offtake, and compounds having boiling points lower than that of trimethylolpropane are taken off at the top or in the upper region of the upper combining column and compounds which have boiling points higher than that of trimethylolpropane are taken off at the bottom or in the lower region of the lower combining column.

2. The process according to claim 1, wherein the reaction mixture has been obtained by reaction of n-butyraldehyde with formaldehyde in the presence of a catalytic amount of a tertiary amine and subsequent hydrogenation.

3. The process according to claim 1 or 2, wherein the major part of the water comprised is removed from the reaction mixture before this reaction mixture is introduced into the feed column.

4. The process according to claim 3, wherein the dewatered reaction mixture is admixed with an alcohol other than trimethylolpropane before this reaction mixture is introduced into the feed column.

5. The process according to any of the preceding claims, wherein the feed column and the offtake column are configured as chambers which are open to a combining zone at each end and extend over part of the longitudinal dimension of a column and are separated from one another by a dividing wall.

6. The process according to any of claims 1 to 4, wherein the offtake column, the upper combining column and the lower combining column are configured as a one-piece distillation column and the feed column is configured as a thermally coupled precolumn before the distillation column.

7. The process according to any of claims 1 to 4, wherein the feed column, the upper combining column and the lower combining column are configured as a one-piece distillation column and the offtake column is configured as a thermally coupled after-column connected to the distillation column.

8. The process according to any of the preceding claims, wherein the feed column, the upper combining column, the lower combining column and the offtake column contain separation-active internals in the form of ordered packing and/or random beds of packing elements.

9. The process according to any of the preceding claims, wherein the distillation is carried out at a pressure in the range from 5 to 100 mbar.

## Revendications

1. Procédé d'isolement de triméthylolpropane à partir d'un mélange réactionnel, qui est obtenu par réaction de n-butyraldéhyde avec du formaldéhyde en présence d'une base et éventuellement hydrogénation, par distillation, dans lequel
i) on introduit le mélange dans une colonne d'entrée comportant une partie de concentration située au-dessus de l'emplacement d'entrée et une partie de rectification située en dessous de l'emplacement d'entrée,
ii) on prévoit une colonne de réunion supérieure qui communique avec l'extrémité supérieure de la partie de concentration et qui comporte un condenseur à l'extrémité supérieure de la colonne et une colonne de réunion inférieure qui communique avec l'extrémité inférieure de la partie de rectification et qui comporte un dispositif de chauffage à l'extrémité inférieure de la colonne,
iii) on prévoit une colonne de soutirage qui communique avec la colonne de réunion supérieure et la colonne de réunion inférieure,
iv) on soutire de la colonne de soutirage du triméthylolpropane pur sous la forme d'un soutirage latéral, et on soutire, à la tête ou dans une zone supérieure de la colonne de réunion supérieure, des composés à point d'ébullition plus bas que le triméthylolpropane et, dans le fond ou une zone inférieure de la colonne de réunion inférieure, des composés à point d'ébullition supérieur au triméthylolpropane.

2. Procédé suivant la revendication 1, dans lequel le mélange réactionnel est obtenu par réaction de n-butyraldéhyde avec du formaldéhyde en présence d'une quantité catalytique d'une amine tertiaire et par hydrogénation ultérieure.

3. Procédé suivant la revendication 1 ou 2, dans lequel, avant l'introduction dans la colonne d'entrée, on élimine du mélange réactionnel la quantité principale d'eau contenue.

4. Procédé suivant la revendication 3, dans lequel, avant l'introduction dans la colonne d'entrée, on ajoute au mélange réactionnel déshydraté un alcool différent du triméthylolpropane.

5. Procédé suivant l'une des revendications précédentes, dans lequel la colonne d'entrée et la colonne de soutirage sont réalisées sous la forme de chambres partielles qui s'étendent sur une section de l'extension longitudinale d'une colonne, sont ouvertes des deux côtés chaque fois vers un compartiment de réunion et sont séparées l'une de l'autre par une paroi séparatrice.

6. Procédé suivant les revendications 1 à 4, dans lequel la colonne de soutirage, la colonne de réunion supérieure et la colonne de réunion inférieure sont réalisées sous la forme d'une colonne de distillation d'une pièce et la colonne d'entrée est réalisée sous la forme d'une colonne préalable thermiquement couplée à la colonne de distillation.

7. Procédé suivant les revendications 1 à 4, dans lequel la colonne d'entrée, la colonne de réunion supérieure et la colonne de réunion inférieure sont réalisées sous la forme d'une colonne de distillation d'une pièce et la colonne de soutirage est réalisée sous la forme d'une colonne postérieure thermiquement couplée à la colonne de distillation.

8. Procédé suivant l'une des revendications précédentes, dans lequel la colonne d'entrée, la colonne de réunion supérieure, la colonne de réunion inférieure et la colonne de soutirage contiennent des éléments encastrés à effet de séparation sous la forme de garnissages ordonnés et/ou d'empilements irréguliers de corps de remplissage.

9. Procédé suivant l'une des revendications précédentes, dans lequel on distille à une pression de l'ordre de 5 à 100 mbars.
